# EUROPEAN PATENT APPLICATION

(11) **EP 4 353 722 A1**
(43) Date of publication of application: **17.04.2024**
(21) Application number: 22815353.2
(22) Date of filing: 02.06.2022
(51) Int. Cl.: C07D 471/04, A61K 31/496, A61P 35/00

(54) **PHOSPHATE OF TRIFLUOROMETHYL-SUBSTITUTED SULFONAMIDE COMPOUND**

(30) Priority: 04.06.2021 CN 202110623940
(71) Applicant: CHIA TAI TIANQING PHARMACEUTICAL GROUP CO., LTD., Lianyungang, Jiangsu 222062 (CN)
(72) Inventor: WANG, Bin, Lianyungang, Jiangsu 222062 (CN); LIU, Fei, Lianyungang, Jiangsu 222062 (CN); FENG, Weiwei, Lianyungang, Jiangsu 222062 (CN)
(74) Representative: Thoma, Michael
(86) International application number: PCT/CN2022/096845
(87) International publication number: WO 2022/253313

(57) **Abstract**

A phosphate compound of a trifluoromethyl-substituted sulfonamide compound for selectively inhibiting anti-apoptotic protein BCL-2, a preparation method therefor, a pharmaceutical composition containing the compound, and the use thereof in the treatment of anti-apoptotic protein BCL-2-related diseases, such as cancer.

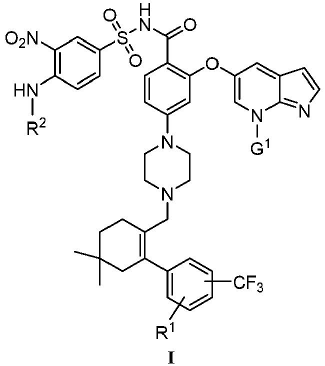

## Description

### CROSS-REFERENCE TO RELATED APPLICATIONS

The present application claims priority and benefit to the Chinese Patent No. 202110623940.6 filed with the National Intellectual Property Administration, PRC on June 4, 2021, which is incorporated herein by reference in its entirety.

### TECHNICAL FIELD

The present application relates to a phosphate compound of a trifluoromethyl-substituted sulfonamide compound that selectively inhibits anti-apoptotic protein BCL-2, a method for preparing same, a pharmaceutical composition containing same, and use thereof for treating an anti-apoptotic protein BCL-2-related disease, such as a cancer.

### BACKGROUND

BCL-2 proteins are classified into three families: BCL-2 family (including members such as BCL-2 and BCL-XL), BAX family and BH3-only family. Among them, the BCL-2 family plays an anti-apoptotic role, while members of the other two families play a pro-apoptotic role.

Anti-apoptotic proteins of the BCL-2 family are associated with many diseases and are being investigated as potential targets of therapeutic agents. Such targets for interventional therapy include, for example, proteins BCL-2 and BCL-XL of the BCL-2 family, *etc.* Recently, inhibitors for proteins of the BCL-2 family have been reported in WO2012071374, WO2010138588, and WO2010065865. Although inhibitors that bind to a target protein with high affinity are introduced therein, binding affinity of compounds is only one of many parameters to be considered. One objective is to produce a compound that preferentially binds to, *i.e.,* has selectivity for, one protein over another. The manifestation of this selectivity, as is well known, is high binding affinity for a specific protein and lower binding affinity for another.

The present application includes a series of compounds that exhibit higher selectivity for anti-apoptotic BCL-2 and BCL-XL proteins, and also have better performance in inhibiting the activity of anti-apoptotic BCL-2 protein. Meanwhile, these compounds have better stability in liver microsomes and optimized pharmacokinetic parameters.

### SUMMARY

In one aspect, the present application relates to a compound of formula I, a stereoisomer thereof or a pharmaceutically acceptable salt thereof: 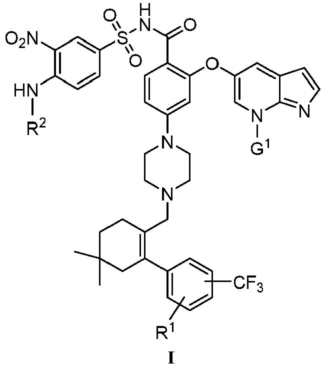 wherein
R¹ is selected from the group consisting of hydrogen, halogen, and C₁₋₃ alkyl;
R² is selected from the group consisting of -R³ and -C₁₋₆ alkylene-R³;
R³ is selected from 5- to 6-membered heterocycloalkyl, wherein the 5- to 6-membered heterocycloalkyl is optionally substituted with one or two groups selected from the group consisting of 4- to 6-membered heterocycloalkyl, C₃₋₆ cycloalkyl, -COR^{a}, -SO₂R^{b}, -COOC₁₋₆ alkyl, and C₁₋₆ alkyl optionally substituted with halogen;
each R^{a} or R^{b} is independently selected from the group consisting of H, 4- to 6-membered heterocycloalkyl, C₃₋₆ cycloalkyl, and C₁₋₆ alkyl, wherein the C₁₋₆ alkyl is optionally substituted with halogen, -CN, -N(C₁₋₆ alkyl)₂, -NHC₁₋₆ alkyl, or -OC₁₋₆ alkyl; and
G¹ is selected from C₁₋₁₀ alkyl substituted with OP(O)(OH)₂.

In some embodiments, the structural fragment 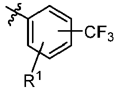 is selected from 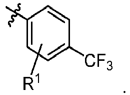 In some embodiments, the structural fragment 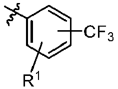 is selected from 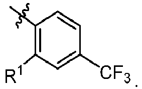 In some embodiments, the structural fragment 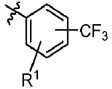 is selected from the group consisting of 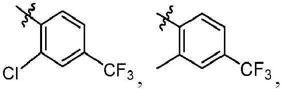 and

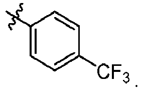

In some embodiments, R¹ is selected from the group consisting of hydrogen, fluorine, chlorine, and C₁₋₃ alkyl. In some embodiments, R¹ is selected from the group consisting of hydrogen, chlorine, and methyl.

In some embodiments, R² is selected from the group consisting of -R³ and -C₁₋₄ alkylene-R³.

In some embodiments, R² is selected from the group consisting of -R³ and -C₁₋₃ alkylene-R³.

In some embodiments, R² is selected from the group consisting of -R³, -CH₂R³, -(CH₂)₂R³, and -(CH₂)₃R³.

In some embodiments, R³ is selected from 5- to 6-membered heterocycloalkyl, wherein the heteroatom in the 5- to 6-membered heterocycloalkyl is selected from the group consisting of N and O, and the number of the heteroatom is selected from the group consisting of 1 and 2.

In some embodiments, R³ is selected from 5- to 6-membered heterocycloalkyl, wherein the 5- to 6-membered heterocycloalkyl is optionally substituted with one or two groups at the ring N atom.

In some embodiments, R³ is selected from 5- to 6-membered heterocycloalkyl, wherein the 5- to 6-membered heterocycloalkyl is optionally substituted with one or two groups selected from the group consisting of 4- to 5-membered heterocycloalkyl, C₄₋₅ alkyl, -COR^{a}, -SO₂R^{b}, -COOC₁₋₃ alkyl, and C₁₋₃ alkyl optionally substituted with halogen.

In some embodiments, R³ is selected from 5- to 6-membered heterocycloalkyl, wherein the 5- to 6-membered heterocycloalkyl is optionally substituted with one or two groups selected from the group consisting of -COR^{a} and SO₂R^{b}.

In some embodiments, each R^{a} or R^{b} is independently selected from the group consisting of 4- to 6-membered heterocycloalkyl, C₃₋₅ cycloalkyl, and C₁₋₅ alkyl, wherein the C₁₋₅ alkyl is optionally substituted with halogen, -CN, -N(C₁₋₃ alkyl)₂, -NHC₁₋₃ alkyl, or -OC₁₋₃ alkyl.

In some embodiments, each R^{a} or R^{b} is independently selected from the group consisting of C₃₋₄ cycloalkyl and C₁₋₄ alkyl, wherein the C₁₋₄ alkyl is optionally substituted with fluorine, chlorine, -CN, -N(C₁₋₃ alkyl)₂, or -OC₁₋₃ alkyl.

In some embodiments, each R^{a} or R^{b} is independently selected from the group consisting of methyl, ethyl, -CH₂CN, cyclopropyl, cyclobutyl, tert-butyl, -CF₃, isopropyl, -CH₂OCH₃, and -CH₂N(CH₃)₂.

In some embodiments, R^{a} is selected from the group consisting of -CH₂CN, cyclopropyl, tert-butyl, -CF₃, isopropyl, -CH₂OCH₃, -CH₂N(CH₃)₂, and methyl.

In some embodiments, R^{b} is selected from the group consisting of methyl, ethyl, cyclopropyl, and cyclobutyl.

In some embodiments, R³ is selected from 5- to 6-membered heterocycloalkyl, wherein the 5- to 6-membered heterocycloalkyl is optionally substituted with -COCH₂CN, -CO-cyclopropane, -COC(CH₃)₃, -COCF₃, -COCH(CH₃)₂, -COCH₂OCH₃, -SO₂CH₃, -SO₂CH₂CH₃, -SO₂-cyclopropane, -SO₂-cyclobutane, -COCH₂N(CH₃)₂, or -COCH₃.

In some embodiments, R³ is selected from the group consisting of tetrahydrofuranyl, tetrahydropyranyl, piperidinyl, morpholinyl, and dioxanyl, wherein the tetrahydrofuranyl, tetrahydropyranyl, piperidinyl, morpholinyl or dioxanyl is optionally substituted with -COCH₂CN, -CO-cyclopropane, -COC(CH₃)₃, -COCF₃, -COCH(CH₃)₂, -COCH₂OCH₃, -SO₂CH₃, -SO₂CH₂CH₃, -SO₂-cyclopropane, -SO₂-cyclobutane, -COCH₂N(CH₃)₂, or -COCH₃.

In some embodiments, R³ is selected from the group consisting of tetrahydrofuranyl, tetrahydropyranyl, and dioxanyl.

In some embodiments, R³ is selected from dioxanyl.

In some embodiments, R³ is selected from the group consisting of piperidinyl and morpholinyl, wherein the piperidinyl or morpholinyl is optionally substituted with -COCH₂CN, -CO-cyclopropane, -COC(CH₃)₃, -COCF₃, -COCH(CH₃)₂, -COCH₂OCH₃, -SO₂CH₃, -SO₂CH₂CH₃, -SO₂-cyclopropane, -SO₂-cyclobutane, -COCH₂N(CH₃)₂, or -COCH₃.

In some embodiments, R³ is selected from the group consisting of

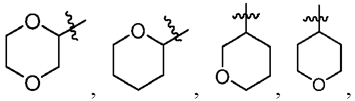

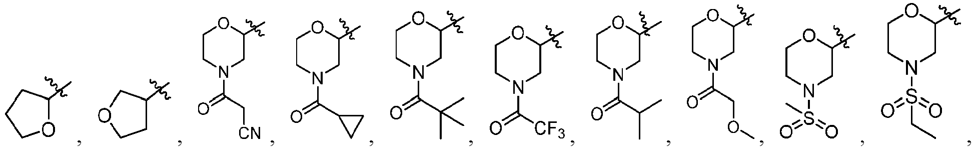

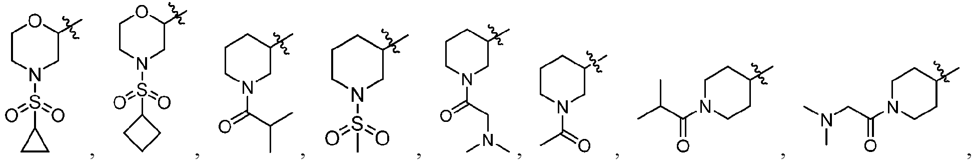

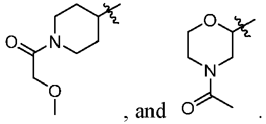

In some embodiments, R³ is selected from the group consisting of 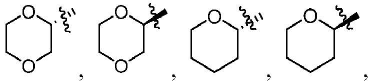 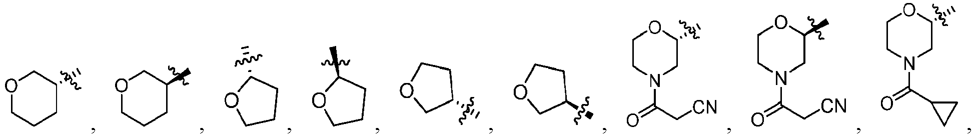 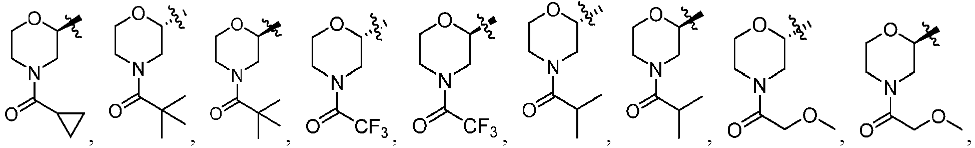 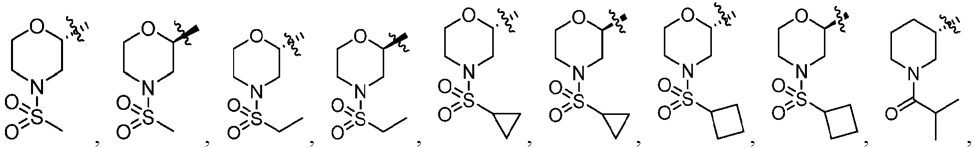 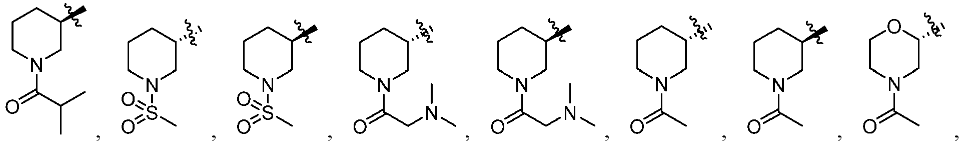 and

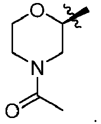

In some embodiments, the heteroatom in the 5- to 6-membered heterocycloalkyl is selected from the group consisting of N and O, and the number of the heteroatom is selected from the group consisting of 1 and 2.

In some embodiments, the 5- to 6-membered heterocycloalkyl is selected from the group consisting of dioxanyl, tetrahydropyranyl, morpholinyl, piperidinyl, and tetrahydrofuranyl.

In some embodiments, the 5- to 6-membered heterocycloalkyl is selected from 6-membered heterocycloalkyl.

In some embodiments, the 5- to 6-membered heterocycloalkyl is selected from the group consisting of dioxanyl, morpholinyl, tetrahydropyranyl, and piperidinyl.

In some embodiments, the 5- to 6-membered heterocycloalkyl is selected from dioxanyl.

In some embodiments, the 5- to 6-membered heterocycloalkyl is selected from morpholinyl.

In some embodiments, the 5- to 6-membered heterocycloalkyl is selected from tetrahydropyranyl.

In some embodiments, the heteroatom in the 4- to 6-membered heterocycloalkyl is selected from the group consisting of N and O, and the number of the heteroatom is selected from the group consisting of 1 and 2. In some embodiments, the heteroatom in the 4- to 6-membered heterocycloalkyl is selected from O, and the number of the heteroatom is 1 or 2. In some embodiments, the 4- to 6-membered heterocycloalkyl is selected from the group consisting of 4-membered heterocycloalkyl, 5-membered heterocycloalkyl, and 6-membered heterocycloalkyl.

In some embodiments, G¹ is selected from C₁₋₆ alkyl substituted with OP(O)(OH)₂.

In some embodiments, G¹ is selected from C₁₋₄ alkyl substituted with OP(O)(OH)₂.

In some embodiments, G¹ is selected from C₁₋₃ alkyl substituted with OP(O)(OH)₂.

In some embodiments, G¹ is selected from -CH₂OP(O)(OH)₂.

In another aspect, the present application relates to a compound of formula II or formula III, a stereoisomer thereof or a pharmaceutically acceptable salt thereof: 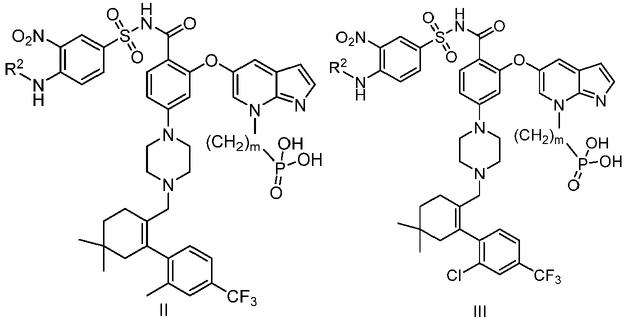 wherein R² is defined as in the compound of formula I; and m is selected from the group consisting of 1, 2, 3, and 4.

The present application relates to a compound of a formula selected from the group consisting of the following formulas, a stereoisomer thereof or a pharmaceutically acceptable salt thereof:

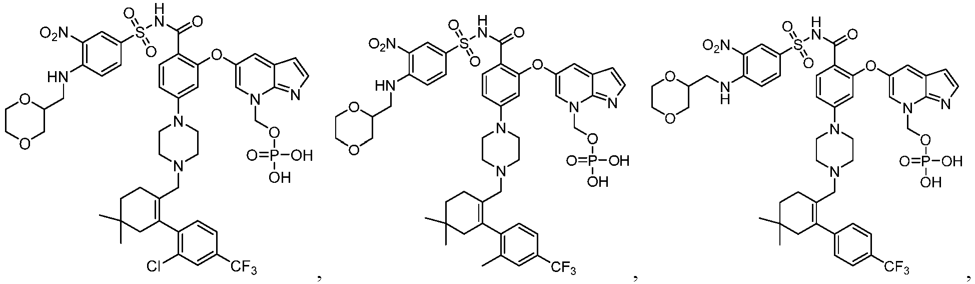

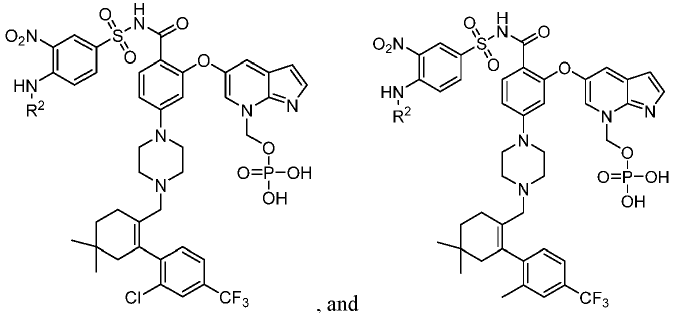

| **R²** | **R²** | **R²** | **R²** | **R²** | **R²** |
|---|---|---|---|---|---|
| 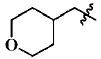 | 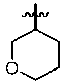 | 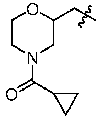 | 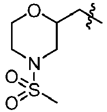 | 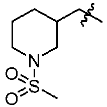 | 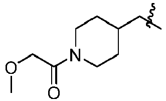 |
| 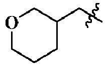 | 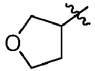 | 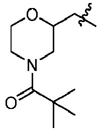 | 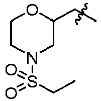 | 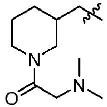 | 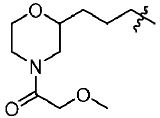 |
| 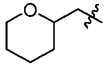 | 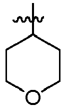 | 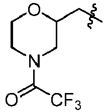 | 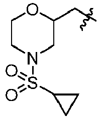 | 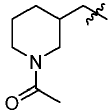 | 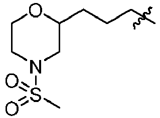 |
| 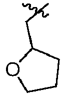 | 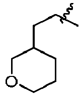 | 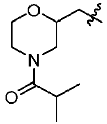 | 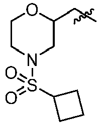 | 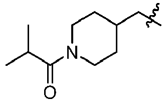 | 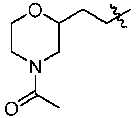 |
| 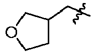 | 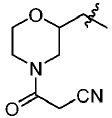 | 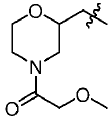 | 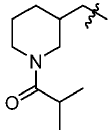 | 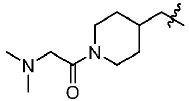 | |

The present application relates to a compound of a formula selected from the group consisting of the following formulas, a stereoisomer thereof or a pharmaceutically acceptable salt thereof:

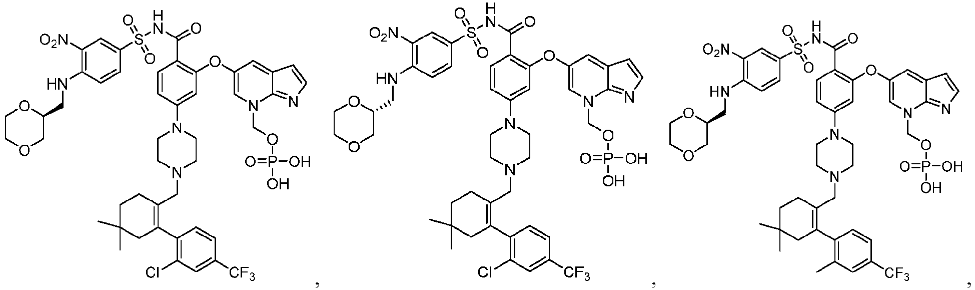

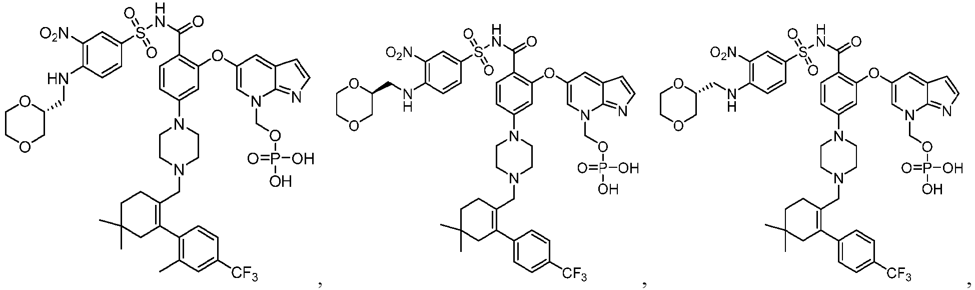

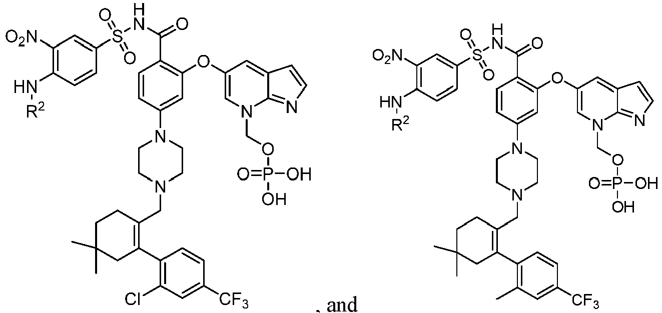

| **R²** | **R²** | **R²** | **R²** | **R²** | **R²** | **R²** | **R²** |
|---|---|---|---|---|---|---|---|
| 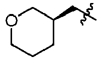 | 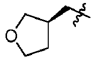 | 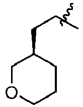 | 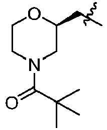 | 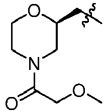 | 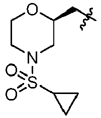 | 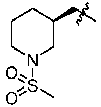 | 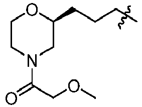 |
| 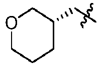 | 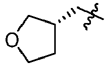 | 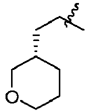 | 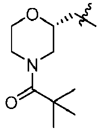 | 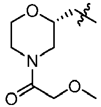 | 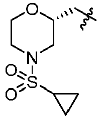 | 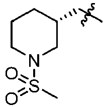 | 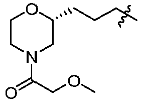 |
| 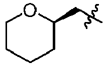 | 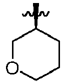 | 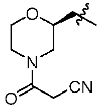 | 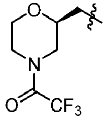 | 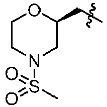 | 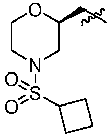 | 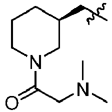 | 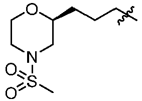 |
| 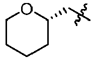 | 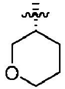 | 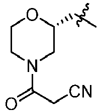 | 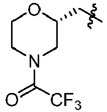 | 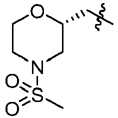 | 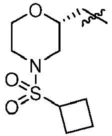 | 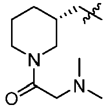 | 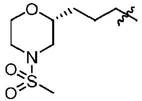 |
| 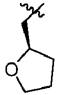 | 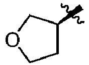 | 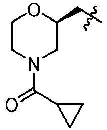 | 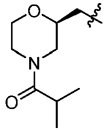 | 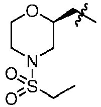 | 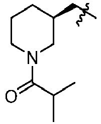 | 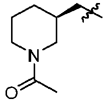 | 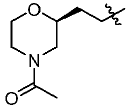 |
| 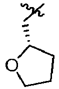 | 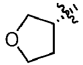 | 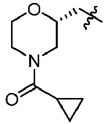 | 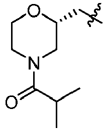 | 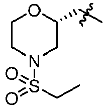 | 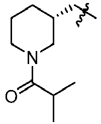 | 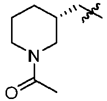 | 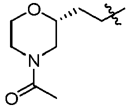 |

The present application relates to a compound of a formula selected from the group consisting of the following formulas, a stereoisomer thereof or a pharmaceutically acceptable salt thereof: 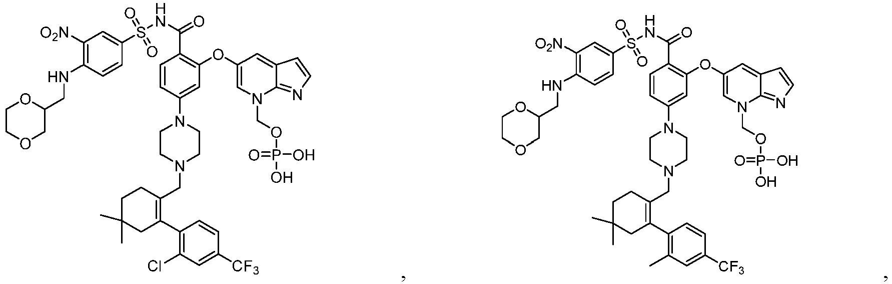 and 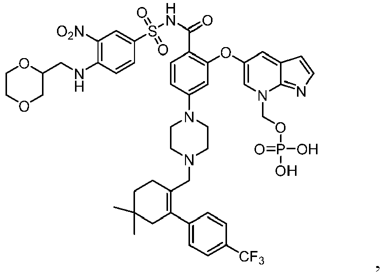 preferably 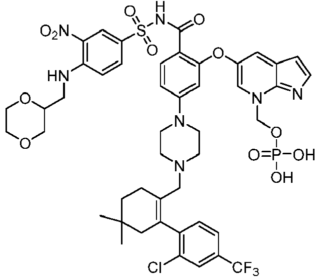 and

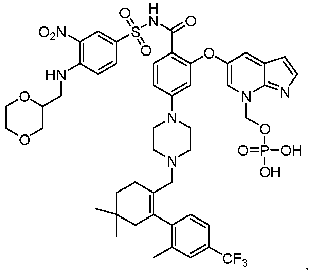

The present application relates to a compound of a formula selected from the group consisting of the following formulas, a stereoisomer thereof or a pharmaceutically acceptable salt thereof: 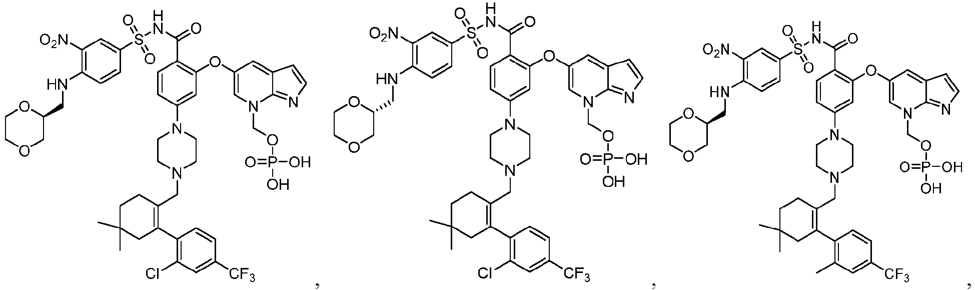 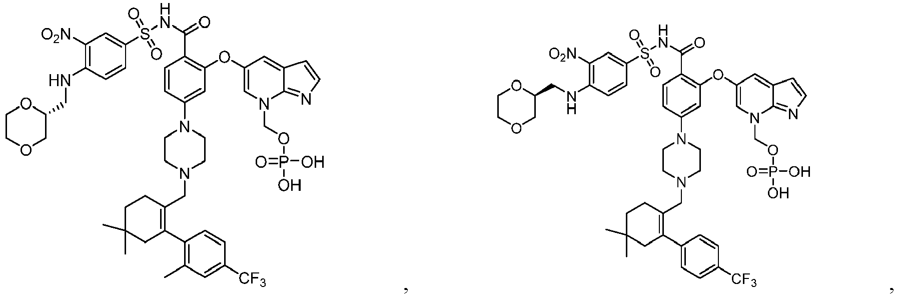 and 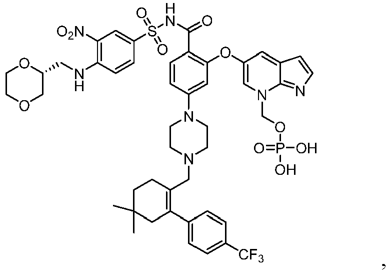 preferably 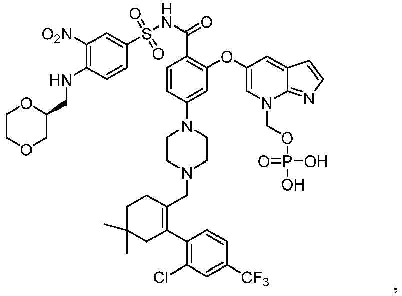 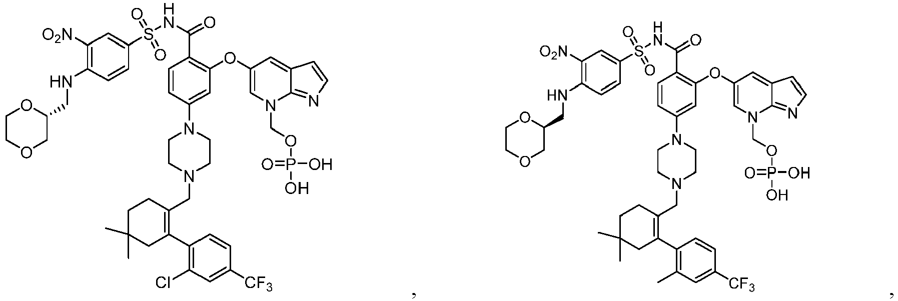 and

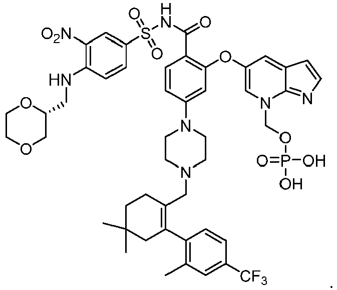

In another aspect, the present application relates to a pharmaceutical composition comprising the compound of formula I, the stereoisomer thereof or the pharmaceutically acceptable salt thereof disclosed herein. In some embodiments, the present application relates to a pharmaceutical composition comprising the compound of formula II or formula III, the stereoisomer thereof or the pharmaceutically acceptable salt thereof disclosed herein. In some embodiments, the pharmaceutical composition of the present application further comprises a pharmaceutically acceptable excipient.

In another aspect, the present application describes a method for treating an anti-apoptotic protein BCL-2-related disease in a mammal, comprising administering to a mammal (preferably a human) in need of such treatment a therapeutically effective amount of the compound, the stereoisomer thereof or the pharmaceutically acceptable salt thereof, or the pharmaceutical composition thereof disclosed herein.

In another aspect, the present application describes use of the compound of formula I, the stereoisomer thereof or the pharmaceutically acceptable salt thereof, or the pharmaceutical composition thereof disclosed herein for preparing a medicament for preventing or treating an anti-apoptotic protein BCL-2-related disease.

In another aspect, the present application describes use of the compound of formula I, the stereoisomer thereof or the pharmaceutically acceptable salt thereof, or the pharmaceutical composition thereof disclosed herein for preventing or treating an anti-apoptotic protein BCL-2-related disease.

In another aspect, the present application describes the compound of formula I, the stereoisomer thereof or the pharmaceutically acceptable salt thereof, or the pharmaceutical composition thereof for use in preventing or treating an anti-apoptotic protein BCL-2-related disease.

The anti-apoptotic protein BCL-2-related disease is selected from a cancer. The cancer is selected from acute lymphocytic leukemia.

The compounds disclosed herein (preferably a specific compound), at the enzyme level, selectively inhibit the BCL-2/BAK enzyme relative to the BCL-XL/BAK enzyme, having lower inhibitory activity for the BCL-XL/BAK enzyme. Moreover, these compounds have good physicochemical properties such as high solubility in water, good *in vivo* and *in vitro* pharmacokinetic properties (such as high bioavailability and high stability before entering into plasma), and good *in vivo* pharmacodynamic properties.

### Definitions

Unless otherwise stated, the following terms used in the present application shall have the following meanings. A certain term, unless otherwise specifically defined, should not be considered uncertain or unclear, but construed according to its common meaning in the field. When referring to a trade name, it is intended to refer to its corresponding commercial product or its active ingredient.

The term "substituted" means that any one or more hydrogen atoms on a specific atom are substituted by substituents, as long as the valence of the specific atom is normal and the resulting compound is stable. When the substituent is oxo (*i.e.,* =O), it means that two hydrogen atoms are substituted and oxo is not available on an aromatic group.

The terms "optional" or "optionally" means that the subsequently described event or circumstance may, but does not necessarily, occur. The description includes instances where the event or circumstance occurs and instances where it does not. For example, ethyl being "optionally" substituted with halogen means that the ethyl may be unsubstituted (-CH₂CH₃), monosubstituted (for example, -CH₂CH₂F), polysubstituted (for example, -CHFCH₂F, -CH₂CHF₂, and the like), or fully substituted (-CF₂CF₃). It will be appreciated by those skilled in the art that for any groups comprising one or more substituents, any substitutions or substituting patterns which may not exist or cannot be synthesized spatially are not introduced.

Cₘ₋ₙ as used herein means that the moiety has an integer number of carbon atoms in the given range. For example, "C₁₋₆" means that the group may have 1 carbon atom, 2 carbon atoms, 3 carbon atoms, 4 carbon atoms, 5 carbon atoms, or 6 carbon atoms.

The term "halo-" or "halogen" refers to fluorine, chlorine, bromine, and iodine.

The term "alkyl" refers to hydrocarbyl with a general formula of CₙH₂ₙ₊₁. The alkyl may be linear or branched. For example, the term "C₁₋₆ alkyl" refers to alkyl containing 1 to 6 carbon atoms (for example, methyl, ethyl, n-propyl, isopropyl, n-butyl, isobutyl, sec-butyl, tert-butyl, n-pentyl, 1-methylbutyl, 2-methylbutyl, 3-methylbutyl, neopentyl, hexyl, 2-methylpentyl, and the like). The alkyl moieties (*i.e.,* alkyl) of alkoxy, alkylamino, dialkylamino, alkylsulfonyl, and alkylthio are similarly defined as above. For another example, the term "C₁₋₄ alkyl" refers to alkyl containing 1 to 4 carbon atoms (for example, methyl, ethyl, *n*-propyl, isopropyl, *n*-butyl, isobutyl, *sec*-butyl, *tert*-butyl, and the like).

The term "alkylene" refers to a divalent group formed by the removal of 1 hydrogen at any position of alkyl. For example, non-limiting examples of the term "C₁₋₆ alkylene" include, but are not limited to, methylene, ethylidene, methylmethylene, dimethylmethylene, 1,3-propylidene, and the like.

The term "cycloalkyl" refers to a carbon ring that is fully saturated and may exist as a monocyclic, bridged cyclic or spiro cyclic structure. Unless otherwise specified, the carbon ring is generally a 3- to 10-membered ring, preferably a 3- to 6-membered ring, such as a 3- to 5-membered ring or a 3- to 4-membered ring. Non-limiting examples of cycloalkyl include, but are not limited to, cyclopropyl, cyclobutyl, cyclopentyl, cyclohexyl, norbornyl(bicyclo[2.2.1]heptyl), bicyclo[2.2.2]octyl, adamantyl, and the like.

The term "heterocycloalkyl" refers to a fully saturated cyclic group which may exist in the form of a monocyclic, bridged cyclic or spiro cyclic structure. Unless otherwise specified, the heterocyclyl is generally a 3- to 7-membered ring (for example, a 4- to 6-membered ring or a 5- to 6-membered ring) containing 1 to 3 heteroatoms (preferably 1 or 2 heteroatoms) independently selected from the group consisting of sulfur, oxygen, and/or nitrogen. Examples of 3-membered heterocycloalkyl include, but are not limited to, oxiranyl, thiiranyl, and aziranyl. Non-limiting examples of 4-membered heterocycloalkyl include, but are not limited to, azetidinyl, oxetanyl, and thietanyl. Examples of 5-membered heterocycloalkyl include, but are not limited to, tetrahydrofuranyl, tetrahydrothienyl, pyrrolidinyl, isoxazolidinyl, oxazolidinyl, isothiazolidinyl, thiazolidinyl, imidazolidinyl, and tetrahydropyrazolyl. Examples of 6-membered heterocycloalkyl include, but are not limited to, piperidinyl, tetrahydropyranyl, tetrahydrothiopyranyl, morpholinyl, piperazinyl, 1,4-oxathianyl, 1,4-dioxanyl, thiomorpholinyl, 1,3-dithianyl, and 1,4-dithianyl. Examples of 7-membered heterocycloalkyl include, but are not limited to, azacycloheptanyl, oxacycloheptanyl, and thiocycloheptanyl. Monocyclic heterocycloalkyl having 5 or 6 ring atoms is preferred.

The term "treat" or "treatment" means administering a compound or formulation of the present application to ameliorate or eliminate a disease or one or more symptoms associated with the disease, including:
(i) inhibiting a disease or disease state, *i.e.,* arresting its development; and
(ii) alleviating a disease or disease state, *i.e.,* causing its regression.

The term "prevent" or "prevention" means administering a compound or formulation of the present application to prevent a disease or one or more symptoms associated with the disease, including: preventing the occurrence of the disease or disease state in a mammal, particularly when such a mammal is predisposed to the disease state but has not yet been diagnosed with it.

The term "effective amount" refers to an amount of the compound disclosed herein for (i) treating or preventing a specific disease, condition or disorder; (ii) alleviating, improving or eliminating one or more symptoms of a specific disease, condition or disorder, or (iii) preventing or delaying onset of one or more symptoms of a specific disease, condition or disorder. The amount of the compound of the present application composing the "therapeutically effective amount" varies depending on the compound, the disease state and its severity, the administration regimen, and the age of the mammal to be treated, but can be determined routinely by those skilled in the art in accordance with their knowledge and the present disclosure.

The term "pharmaceutically acceptable" is used herein for those compounds, materials, compositions, and/or dosage forms which are, within the scope of sound medical judgment, suitable for use in contact with the tissues of human beings and animals without excessive toxicity, irritation, allergic response, or other problems or complications, and commensurate with a reasonable benefit/risk ratio.

A pharmaceutically acceptable salt, for example, may be a metal salt, an ammonium salt, a salt formed with an organic base, a salt formed with an inorganic acid, a salt formed with an organic acid, a salt formed with a basic or acidic amino acid, and the like.

The term "pharmaceutical composition" refers to a mixture consisting of one or more of the compounds or the salts thereof of the present application and a pharmaceutically acceptable excipient. The pharmaceutical composition is intended to facilitate the administration of the compound of the present application to an organism. The term "pharmaceutically acceptable excipient" refers to those that do not have a significant irritating effect on an organism and do not impair the biological activity and properties of the active compound. Suitable excipients are well known to those skilled in the art, for example, carbohydrate, wax, water-soluble and/or water-swellable polymers, hydrophilic or hydrophobic material, gelatin, oil, solvent, or water.

The word "comprise" and variations thereof such as "comprises" or "comprising" will be understood in an open, non-exclusive sense, *i.e.,* "including but not limited to".

The compounds and intermediates of the present application may also exist in different tautomeric forms, and all such forms are included within the scope of the present application. The term "tautomer" or "tautomeric form" refers to structural isomers of different energies that can interconvert via a low energy barrier. For example, a proton tautomer (also referred to as prototropic tautomer) includes interconversion via proton transfer, such as keto-enol isomerism and imine-enamine isomerization. A specific example of a proton tautomer is an imidazole moiety where a proton can transfer between two ring nitrogens. A valence tautomer includes the interconversion via recombination of some bonding electrons.

The present application also comprises isotopically labeled compounds of the present application which are identical to those recited herein but have one or more atoms replaced by an atom having an atomic mass or mass number different from the atomic mass or mass number generally found in nature. Examples of isotopes that can be incorporated into the compounds of the present application include isotopes of hydrogen, carbon, nitrogen, oxygen, phosphorus, sulfur, fluorine, iodine and chlorine, such as ²H, ³H, ¹¹C, ¹³C, ¹⁴C, ¹³N, ¹⁵N, ¹⁵O, ¹⁷O, ¹⁸O, ³¹P, ³²P, ³⁵S, ¹⁸F, ¹²³I, ¹²⁵I, and ³⁶Cl.

Certain isotopically labeled compounds of the present application (*e.g*., those labeled with ³H and ¹⁴C) can be used to analyze compounds and/or substrate tissue distribution. Tritiated (*i.e.,* ³H) and carbon-14 (*i.e.,* ¹⁴C) isotopes are particularly preferred for their ease of preparation and detectability. Positron emitting isotopes, such as ¹⁵O, ¹³N, ¹¹C, and ¹⁸F, can be used in positron emission tomography (PET) studies to determine substrate occupancy. Isotopically labeled compounds of the present application can generally be prepared by following procedures analogous to those disclosed in the schemes and/or examples below while substituting a non-isotopically labeled reagent with an isotopically labeled reagent.

Furthermore, substitution with heavier isotopes such as deuterium (*i.e.,* ²H) may provide certain therapeutic advantages (*e.g.,* increased *in vivo* half-life or reduced dosage requirement) resulting from greater metabolic stability and hence may be preferred in some circumstances in which deuterium substitution may be partial or complete, wherein partial deuterium substitution refers to substitution of at least one hydrogen with at least one deuterium.

The compound of the present application can be asymmetrical, for example, has one or more stereoisomers. Unless otherwise stated, all stereoisomers are included in the present application, such as enantiomers and diastereoisomers. The compound with asymmetrical carbon atoms of the present application can be separated in an optically pure form or in a racemic form. The optically pure form can be separated from a racemic mixture or can be synthesized using a chiral raw material or a chiral reagent.

The pharmaceutical composition of the present application can be prepared by combining the compound of the present application with a suitable pharmaceutically acceptable excipient, and can be formulated, for example, into a solid, semisolid, liquid or gaseous formulation such as tablet, pill, capsule, powder, granule, ointment, emulsion, suspension, suppository, injection, inhalant, gel, microsphere, and aerosol.

Typical routes of administration of the compound or the pharmaceutically acceptable salt thereof or the pharmaceutical composition thereof of the present application include, but are not limited to, oral, rectal, local, inhalation, parenteral, sublingual, intravaginal, intranasal, intraocular, intraperitoneal, intramuscular, subcutaneous and intravenous administration.

The pharmaceutical composition of the present application can be manufactured using methods well known in the art, such as conventional mixing, dissolving, granulating, dragee-making, levigating, emulsifying and lyophilizing.

In some embodiments, the pharmaceutical composition is in an oral form. For oral administration, the pharmaceutical composition can be formulated by mixing the active compounds with pharmaceutically acceptable excipients well known in the art. These excipients enable the compounds of the present application to be formulated into tablets, pills, pastilles, dragees, capsules, liquids, gels, slurries, suspensions, and the like for oral administration to a patient.

A solid oral composition can be prepared by conventional mixing, filling or tableting. For example, it can be obtained by the following method: mixing the active compounds with solid excipients, optionally grinding the resulting mixture, adding additional suitable excipients if desired, and processing the mixture into granules to get the core parts of tablets or dragees. Suitable excipients include, but are not limited to: binders, diluents, disintegrants, lubricants, glidants, sweeteners or flavoring agents, and the like.

The pharmaceutical compositions may also be suitable for parenteral administration, such as sterile solutions, suspensions or lyophilized products in suitable unit dosage forms.

In all of the administration methods of the compound of formula I described herein, the daily dosage administered is from 0.01 mg/kg to 200 mg/kg body weight, given in individual or separated dosages.

The compounds of the present application can be prepared by a variety of synthetic methods well known to those skilled in the art, including the specific embodiments listed below, embodiments formed by combinations thereof with other chemical synthetic methods, and equivalents thereof known to those skilled in the art. The preferred embodiments include, but are not limited to, the examples of the present application. The chemical reactions of the specific embodiments of the present application are conducted in a proper solvent that must be suitable for the chemical changes in the present application and the reagents and materials required. In order to obtain the compounds of the present application, it is sometimes necessary for those skilled in the art to modify or select a synthesis procedure or a reaction process based on the existing embodiments.

An important consideration in synthetic route planning in the art is the selection of suitable protecting groups for reactive functional groups (e.g., hydroxyl in the present application). For example, reference may be made to Greene's Protective Groups in Organic Synthesis (4th Ed.) Hoboken, New Jersey: John Wiley & Sons, Inc.

All patents, patent applications, and other identified publications are explicitly incorporated by reference in the present application for the purpose of description and disclosure. These publications are provided solely because they were disclosed prior to the filing date of the present application. All statements as to the dates of these documents or description as to the contents of these documents are based on the information available to the applicant and do not constitute any admission as to the correctness of the dates or the content of these documents. Moreover, in any country or region, any reference to these publications herein is not to be construed as an admission that the publications form part of the common knowledge in the art.

### DETAILED DESCRIPTION

For clarity, the present application is further described with the following examples, which are, however, not intended to limit the scope of the present application. All reagents used in the present application are commercially available and can be used without further purification.

tBu stands for *tert*-butyl.

### Example 1

### 1) Preparation of compound 1-1

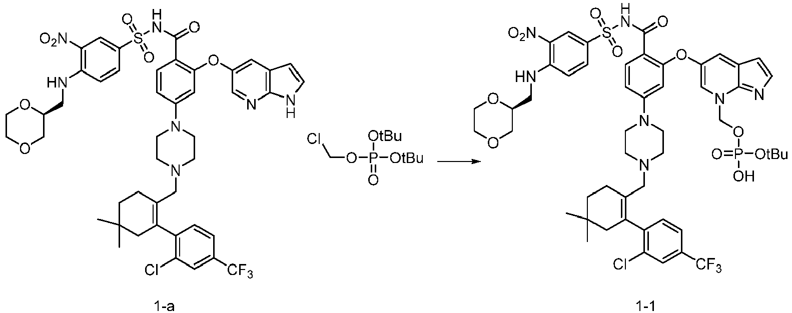

Compound 1-a (2 g, prepared according to Example 8 in WO2020088442), di-tert-butyl chloromethyl phosphate (1.65 g), and 1,8-bisdimethylnaphthalene (0.50 g) were added to acetonitrile (10 mL) and mixed with stirring. The resulting mixture was heated to 80 °C, stirred for 6 h, then naturally cooled to room temperature, stirred for another 12 h, and concentrated under reduced pressure to give compound 1-1. ESI-MS: m/z = 1104.8 [M+H]⁺.

### 2) Preparation of compound 1

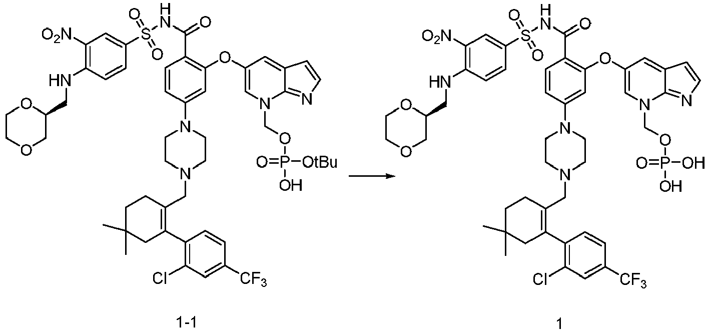

Compound 1-1 (1.9 g) was added to dichloromethane (5 mL), and trifluoroacetic acid (5 mL) was added dropwise. The resulting mixture was stirred at room temperature for 12 h, concentrated under reduced pressure, and then purified by preparative liquid chromatography to give compound 1 (340 mg).

¹H NMR (500 MHz, DMSO-d6), δ: 8.75 (1H, d), 8.60 (1H, t,), 8.55 (1H, d), 8.52 (1H, d), 7.99 (1H, d), 7.89 (1H, m), 7.86 (1H, dd), 7.70 (1H, d), 7.57 (1H, d), 7.39 (1H, d), 7.19 (1H, d), 6.88 (1H, d), 6.81 (1H, dd), 6.43 (1H, d), 6.36 (1H, s), 6.33 (1H, s), 3.81 (1H, m), 3.81 (1H, m), 3.79 (1H, m), 3.67 (1H, m), 3.61 (1H, m), 3.58 (1H, m), 3.53 (1H, m), 3.49 (1H, m), 3.42 (1H, m), 3.41 (1H, m), 3.33 (1H, m), 3.06 (4H, brs), 2.28 (1H, m), 2.19 (1H, m), 2.15 (4H, m), 1.96 (2H, m), 1.49 (2H, m), 0.97 (3H, s), 0.96 (3H, s). ESI-MS: m/z = 1048.8 [M+H]⁺.

### Example 2

### 1) Preparation of compound 2-1

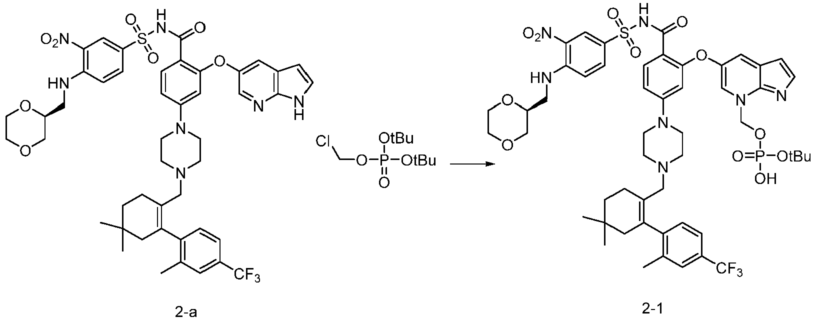

Compound 2-1 was obtained by reference to the preparation method for compound 1-1, with compound 1-a being replaced by compound 2-a (prepared according to Example 3 in WO2020238785). ESI-MS: m/z = 1084.9 [M+H]⁺.

### 2) Preparation of compound 2

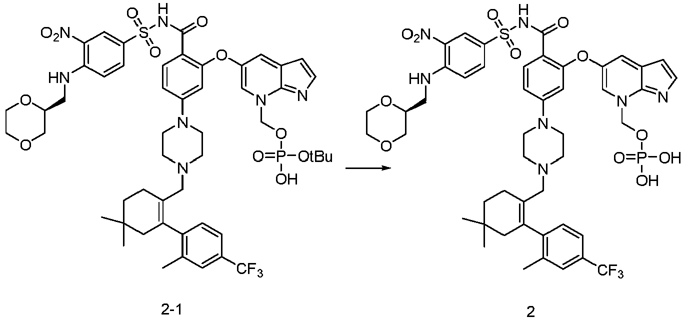

Compound 2 was obtained by reference to the preparation method for compound 1, with compound 1-1 being replaced by compound 2-1.

¹H NMR (500 MHz, DMSO-d6), δ: 8.75 (1H, d), 8.60 (1H, t,), 8.54 (1H, d), 8.51 (1H, d), 7.99 (1H, d), 7.86 (1H, dd), 7.84 (1H, d), 7.57 (1H, d), 7.50 (1H, d), 7.19 (1H, d), 7.17 (1H, d), 6.87 (1H, d), 6.79 (1H, dd), 6.43 (1H, d), 6.34 (1H, s), 6.32 (1H, s), 3.79 (4H, m), 3.64 (4H, m), 3.49 (2H, m), 3.41 (1H, m), 3.29 (4H, m), 3.02 (2H, brs), 2.91 (1H, brs), 2.64 (1H, m), 2.29 (1H, m), 2.18 (1H, m), 2.16 (3H, s), 1.91 (2H, m), 1.49 (2H, m), 0.97 (3H, s), 0.96 (3H, s). ESI-MS: m/z = 1028.6 [M+H]⁺.

### Experimental Example 1. Inhibitory Activity for In Vitro Protein Binding

### 1.1. Screening on inhibitory activity for BCL-2/BAK binding

A 500 nM Tag1-BCL-2 protein stock solution and a 20 µM Tag2-BAK protein stock solution were diluted to 5 nM and 120 nM respectively with a dilution buffer in a kit (manufacturer: Cisbio, Cat. No.: 63ADK000CB01PEH). 5 µL of Tag1-BCL-2 protein diluent was added to each well, and then different compounds dissolved in DMSO were added to the wells using a nanoliter pipettor, allowing the final compound concentrations to be 200 nM to 0.0488 nM (4-fold gradient for 7 concentrations in total). Blank control wells (without enzyme) and negative control wells (with enzyme, plus vehicle DMSO) were set, and 2 replicate wells were set. Finally, 5 µL of Tag2-BAK protein diluent was added to each well, and the mixture was mixed well by centrifugation and incubated at 25 °C for 15 min. 100× anti-Tag1-Eu³⁺ and 100× anti-Tag2-XL665 were both diluted to 1× working concentration with a detection buffer in the kit. Anti-Tag1-Eu³⁺ and anti-Tag2-XL665 were mixed well in a 1:1 ratio, and 5 µL of the mixture was added to each well. The resulting mixture was incubated at 25 °C for 2 h or more. The plate was read using a PE Envision multi-functional microplate reader (excitation: 620 nm, emission: 665 nm), and IC₅₀ (shown in Table 1) was calculated by four-parameter fitting.

### 1.2. Screening on inhibitory activity for BCL-XL/BAK binding

A 300 nM Tag1-BCL-XL protein stock solution and a 10 µM Tag2-BAK protein stock solution were diluted to 2 nM and 80 nM respectively with a dilution buffer in a kit (manufacturer: Cisbio, Cat. No.: 63ADK000CB04PEH). 5 µL of Tag1-BCL-XL protein diluent was added to each well, and then different compounds dissolved in DMSO were added to the wells using a nanoliter pipettor, allowing the final compound concentrations to be 2000 nM to 0.488 nM (4-fold gradient for 7 concentrations in total). Blank control wells (without enzyme) and negative control wells (with enzyme, plus vehicle DMSO) were set, and 2 replicate wells were set. Finally, 5 µL of Tag2-BAK protein diluent was added to each well, and the mixture was mixed well by centrifugation and incubated at 25 °C for 15 min. 100× anti-Tag1-Eu³⁺ and 100× anti-Tag2-XL665 were both diluted to 1× working concentration with a detection buffer in the kit. Anti-Tag1-Eu³⁺ and anti-Tag2-XL665 were mixed well in a 1:1 ratio, and 5 µL of the mixture was added to each well. The resulting mixture was incubated at 25 °C for 2 h or more. The plate was read using a PE Envision multi-functional microplate reader (excitation: 620 nm, emission: 665 nm), and IC₅₀ (shown in Table 1) was calculated by four-parameter fitting.

**Table 1. Inhibitory activity of compounds for BCL-2/BAK and BCL-XL/BAK bindings**

| Compound | BCL-2/BAK | BCL-XL/BAK | Compound | BCL-2/BAK | BCL-XL/BAK |
|---|---|---|---|---|---|
| | IC₅₀ (nM) | | | IC₅₀ (nM) | |
| Example 1 | 3.0 | 328 | Example 2 | 2.9 | 251 |

### Experimental Example 2. Inhibitory Effect of Compounds on Proliferation of RS4;11 Cells

RS4;11 cells in logarithmic growth phase and good cell condition were added to a centrifuge tube and centrifuged at 1500 rpm for 3 min in a low speed centrifuge. The supernatant was discarded, and 5 mL of complete medium (RPMI basic medium + 10% FBS) was added using a pipette for cell resuspension. The cells were counted using a cell counter and diluted with complete medium to a cell density of 2 × 10⁵ cells/mL, and then an equivalent amount of RPMI basic medium was added to adjust the serum concentration to 5% and the cell density to 1 × 10⁵ cells/mL for plate seeding. The cells were seeded on a 96-well plate at 100 µL/well using a multi-channel pipette and cultured in a cell incubator at 37 °C, 5% CO₂ with saturated humidity. After 24 h of incubation, compounds (500-0.1 nM) were loaded using a nanoliter pipettor, 2 replicate wells were set for each concentration, and cells without compound were used as negative controls. After 72 h, CCK-8 was added at 10 µL/well for incubation for 4 h, then absorbance was measured at 450 nm with an Envision plate reader, and inhibition rate was calculated. Inhibition rate (%) = (mean value of negative control group - mean value of experimental group) / (mean value of negative control group - mean value of blank group) × 100%. A dose-response curve was fitted by four-parameter analysis, with the logarithm of compound concentration as abscissa and inhibition rate as ordinate, so that IC₅₀ was calculated (see Table 2).

**Table 2. Inhibitory effect of compounds on proliferation of RS4;11 cells**

| Compound | RS4;11 cells | Compound | RS4;11 cells |
|---|---|---|---|
| | IC₅₀ (nM) | | IC₅₀ (nM) |
| Example 1 | 15.9 | Example 2 | 14.8 |

### Experimental Example 3. In Vitro Intestinal S9 Stability

Intestinal S9 (species: human, monkey, dog, rat, and mouse) incubated samples were prepared by mixing a PBS buffer (pH 7.4), an intestinal S9 solution (1 mg/mL), a test compound, and an NADPH + MgCl₂ solution and incubated at 37 °C and 300 rpm for 2 h. Zero-hour samples were prepared by mixing a PBS buffer (pH 7.4), an intestinal S9 solution (1 mg/mL), and a test compound. An acetonitrile solution containing an internal standard was added to the samples, and supernatants were prepared by protein precipitation, diluted, and then assayed by LC/MS/MS.

Result: The test compounds of the present application have *in vitro* intestinal S9 metabolic stability.

### Experimental Example 4. In Vivo Pharmacokinetics in Rats

SD rats weighing 200-220 g were randomized into groups of 3 after 3-5 days of acclimatization and then separately given the compounds to be tested by intragastric administration at a dose of 10 mg/kg (based on the active ingredient).

Plasma samples to be tested were prepared by taking blood from the orbit at time points of 0.25 (15 min), 0.5 (30 min), 1, 2, 3, 5, 7, 10 and 24 h. 50 µL of each plasma sample to be detected and a standard curve sample were taken, and an acetonitrile solution containing an internal standard was added. A supernatant was obtained by protein precipitation, diluted, and then assayed by LC/MS/MS.

Pharmacokinetic parameters were fitted using a non-compartmental model (see Table 3).

**Table 3. Pharmacokinetic parameters for compounds**

| PK parameter | Example 1 IG | Example 2 IG |
|---|---|---|
| Dose (mg/kg) | 11.2 (based on the active ingredient, 10 mpk) | 11.2 (based on the active ingredient, 10 mpk) |
| Tₘₐₓ (h) | 4.3 ± 1.2 | 4.3 ± 1.2 |
| Cₘₐₓ (ng/mL) | 256 ± 87.6 | 150 ± 52.8 |
| AUC(0-24h) (ng*h/mL) | 2613 ± 1978 | 1505 ± 479 |
| AUC(0-∞) (ng*h/mL) | 3470 ± 2646 | 1826 ± 640 |
| t_{1/2} (h) | 11.1 ± 2.1 | 9.1 ± 0.5 |
| MRT(0-t) (h) | 9.0 ± 1.5 | 8.6 ± 0.5 |

### Experimental Example 5. Determination of Solubility

Reagents used were 0.2 M NaOH (SinoPharm lot No. 20180223) and 0.2 M phosphate (SinoPharm 20180910, pH 7.4). Instruments/equipment used included a balance (HPLC Dionex U3000, Mettler XPE105DR), pipettes (TIANBO 0.5 mL and 0.1 mL), a thermostatic CNC shaker (ZWY-240, set at 25 °C and 200 rpm).

The example compounds were tested in an aqueous medium at 25 °C. An excess of each example compound was weighed out and mixed with an aliquot of the target medium in a clear glass vial. The vial was sealed with a cap and then shaken in the shaker at 25 °C as appropriate. When equilibration was completed, the sample was removed from the shaker and the final pH was measured. The suspension was filtered through a 13 mm needle-type filter and a 0.22 µm Syring Filter needle-type filter (hydrophilic PTFE-SA membrane, lot No. HKS13SATQ2B). Each filter was used for only one sample. The determination was carried out after the filtrate had been suitably diluted with the same solvent as used for the stock solution. The concentration of the sample was calculated based on a calibration curve of the compound. The results are shown in Table 4.

**Table 4. Solubility in water at pH 7.4**

| Example | Solubility in water (pH 7.4, µg/mL) |
|---|---|
| 1 | 19390 |
| 2 | 2230 |

### Experimental Example 6. Pharmacodynamics of Compounds in Human B-Cell Leukemia RS4;11 Subcutaneous Xenograft Model

NOD/SCID mice, female, 9-10 weeks old (ages upon tumor cell grafting), body weight of 16.3-22.0 g, purchased from Beijing AniKeeper Biotech Co., Ltd., with production license number of SCXK (Jing) 2017-0006 and animal certification number of 11402400013155. Housing environment: SPF. Mice were grafted subcutaneously on the right anterior dorsal side with 1 × 10⁷ RS4;11 cells. The day of grafting was defined as day 0. When the mean tumor volume reached 240 mm³, the mice were randomly grouped according to the tumor size. Administration was carried out as in Table 5 below.

**Table 5. Administration route, dosage and regimen for human B-cell leukemia RS4;11 subcutaneous xenograft model**

| Groups | n | Treatment group | Dose (mg/kg body weight) | Mode of administration | Time of administration |
|---|---|---|---|---|---|
| 1 | 6 | Vehicle | | p.o. | Single dose |
| 2 | 6 | **Compound 1** | 25 | p.o. | Single dose |
| 3 | 6 | **Compound 1** | 50 | p.o. | Single dose |
| 4 | 6 | **Compound 2** | 25 | p.o. | Single dose |
| 5 | 6 | **Compound 2** | 50 | p.o. | Single dose |

| | | | | | |
|---|---|---|---|---|---|
| Note: n: the number of the animals; volume of administration: 10 µL/g. | | | | | |

Clinical symptoms observed during the study were recorded in the raw data. Calculation formula for tumor volume: Tumor volume (mm³) = 1/2 × (a × b²) (where a represents long diameter and b represents short diameter). The data were collected using StudyDirector^{™} software (version No. 3.1.399.19, supplier: Studylog System, Inc., S. San Francisco, CA, USA) in the experiment, including measurements of the long and short diameters of the tumor and weighing results of the animals. The raw data obtained from a balance and a vernier caliper were directly input into the software, and any change in the data was recorded. Relative tumor proliferation rate (T/C%) refers to the percentage of the relative tumor volume or tumor weight of the treatment and control groups at a certain time point. The calculation formula is as follows:
T/C% = T_{RTV} / C_{RTV} × 100% (T_{RTV}: mean RTV for treatment group; C_{RTV}: mean RTV for vehicle control group; RTV = Vₜ / V₀, where V₀ denotes the tumor volume of the animal upon grouping, and Vₜ denotes the tumor volume of the animal after treatment).

Tumor growth inhibition rate (TGI%) was calculated according to the following formula: TGI% = (1 - T/C) × 100%. (T and C are the relative tumor volume (RTV) or tumor weight (TW) at a particular time point for the treatment and control groups, respectively).

All experimental results were expressed as mean tumor volume ± SEM (standard error of mean). The relative tumor volume of the treatment group was compared with that of the control group for any significant difference by the independent sample T test. All data were analyzed using SPSS 18.0. p < 0.05 was defined as a significant difference.

The results show that the compounds of the present application can achieve good *in vivo* efficacy.

## Claims

1. A compound of formula I, a stereoisomer thereof or a pharmaceutically acceptable salt thereof: 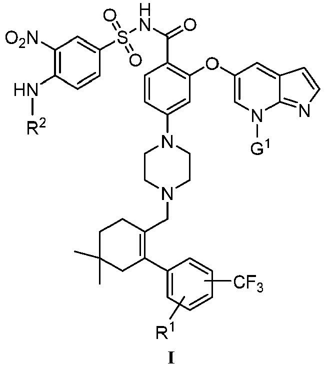 wherein
R¹ is selected from the group consisting of hydrogen, halogen, and C₁₋₃ alkyl;
R² is selected from the group consisting of -R³ and -C₁₋₆ alkylene-R³;
R³ is selected from 5- to 6-membered heterocycloalkyl, wherein the 5- to 6-membered heterocycloalkyl is optionally substituted with one or two groups selected from the group consisting of 4- to 6-membered heterocycloalkyl, C₃₋₆ cycloalkyl, -COR^{a}, -SO₂R^{b}, -COOC₁₋₆ alkyl, and C₁₋₆ alkyl optionally substituted with halogen;
each R^{a} or R^{b} is independently selected from the group consisting of H, 4- to 6-membered heterocycloalkyl, C₃₋₆ cycloalkyl, and C₁₋₆ alkyl, wherein the C₁₋₆ alkyl is optionally substituted with halogen, -CN, -N(C₁₋₆ alkyl)₂, -NHC₁₋₆ alkyl, or -OC₁₋₆ alkyl; and
G¹ is selected from C₁₋₁₀ alkyl substituted with OP(O)(OH)₂.

2. The compound of formula I, the stereoisomer thereof or the pharmaceutically acceptable salt thereof according to claim 1, wherein the structural fragment 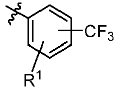 is 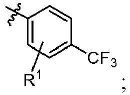 or the structural fragment 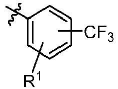 is 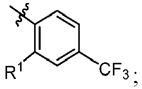 or the structural fragment 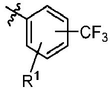 is selected from the group consisting of 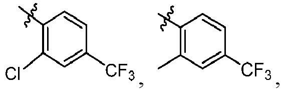 and

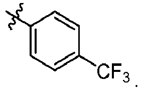

3. The compound of formula I, the stereoisomer thereof or the pharmaceutically acceptable salt thereof according to claim 1 or 2, wherein R¹ is selected from the group consisting of hydrogen, fluorine, chlorine, and C₁₋₃ alkyl;
or R¹ is selected from the group consisting of hydrogen, chlorine, and methyl.

4. The compound of formula I, the stereoisomer thereof or the pharmaceutically acceptable salt thereof according to any one of claims 1 to 3, wherein R² is selected from the group consisting of -R³ and -C₁₋₄ alkylene-R³;
or R² is selected from the group consisting of -R³ and -C₁₋₃ alkylene-R³;
or R² is selected from the group consisting of -R³, -CH₂R³, -(CH₂)2R³, and -(CH₂)₃R³.

5. The compound of formula I, the stereoisomer thereof or the pharmaceutically acceptable salt thereof according to any one of claims 1 to 4, wherein R³ is selected from 5- to 6-membered heterocycloalkyl, wherein the 5- to 6-membered heterocycloalkyl is optionally substituted with one or two groups at an N atom of the heterocycloalkyl ring;
or R³ is selected from 5- to 6-membered heterocycloalkyl, wherein the 5- to 6-membered heterocycloalkyl is optionally substituted with one or two groups selected from the group consisting of 4- to 5-membered heterocycloalkyl, C₄₋₅ alkyl, -COR^{a}, -SO₂R^{b}, -COOC₁₋₃ alkyl, and C₁₋₃ alkyl optionally substituted with halogen;
or R³ is selected from 5- to 6-membered heterocycloalkyl, wherein the 5- to 6-membered heterocycloalkyl is optionally substituted with one or two groups selected from the group consisting of -COR^{a} and SO₂R^{b}.

6. The compound of formula I, the stereoisomer thereof or the pharmaceutically acceptable salt thereof according to any one of claims 1 to 5, wherein each R^{a} or R^{b} is independently selected from the group consisting of 4- to 6-membered heterocycloalkyl, C₃₋₅ cycloalkyl, and C₁₋₅ alkyl, wherein the C₁₋₅ alkyl is optionally substituted with halogen, -CN, -N(C₁₋₃ alkyl)₂, -NHC₁₋₃ alkyl, or -OC₁₋₃ alkyl;
or each R^{a} or R^{b} is independently selected from the group consisting of C₃₋₄ cycloalkyl and C₁₋₄ alkyl, wherein the C₁₋₄ alkyl is optionally substituted with fluorine, chlorine, -CN, -N(C₁₋₃ alkyl)₂, and -OC₁₋₃ alkyl;
or each R^{a} or R^{b} is independently selected from the group consisting of methyl, ethyl, -CH₂CN, cyclopropyl, cyclobutyl, tert-butyl, -CF₃, isopropyl, -CH₂OCH₃, and -CH₂N(CH₃)₂;
or R^{a} is selected from the group consisting of -CH₂CN, cyclopropyl, tert-butyl, -CF₃, isopropyl, -CH₂OCH₃, -CH₂N(CH₃)₂, and methyl;
or R^{b} is selected from the group consisting of methyl, ethyl, cyclopropyl, and cyclobutyl.

7. The compound of formula I, the stereoisomer thereof or the pharmaceutically acceptable salt thereof according to any one of claims 1 to 6, wherein R³ is selected from 5- to 6-membered heterocycloalkyl, wherein the 5- to 6-membered heterocycloalkyl is optionally substituted with -COCH₂CN, -CO-cyclopropane, -COC(CH₃)₃, -COCF₃, -COCH(CH₃)₂, -COCH₂OCH₃, -SO₂CH₃, -SO₂CH₂CH₃, -SO₂-cyclopropane, -SO₂-cyclobutane, -COCH₂N(CH₃)₂, or -COCH₃;
or R³ is selected from the group consisting of tetrahydrofuranyl, tetrahydropyranyl, piperidinyl, morpholinyl, and dioxanyl, wherein the tetrahydrofuranyl, tetrahydropyranyl, piperidinyl, morpholinyl or dioxanyl is optionally substituted with -COCH₂CN, -CO-cyclopropane, -COC(CH₃)₃, -COCF₃, -COCH(CH₃)₂, -COCH₂OCH₃, -SO₂CH₃, -SO₂CH₂CH₃, -SO₂-cyclopropane, -SO₂-cyclobutane, -COCH₂N(CH₃)₂, or -COCH₃;
or R³ is selected from the group consisting of tetrahydrofuranyl, tetrahydropyranyl, and dioxanyl;
or R³ is selected from the group consisting of piperidinyl and morpholinyl, wherein the piperidinyl or morpholinyl is optionally substituted with -COCH₂CN, -CO-cyclopropane, -COC(CH₃)₃, -COCF₃, -COCH(CH₃)₂, -COCH₂OCH₃, -SO₂CH₃, -SO₂CH₂CH₃, -SO₂-cyclopropane, -SO₂-cyclobutane, -COCH₂N(CH₃)₂, or -COCH₃; preferably, R³ is selected from the group consisting of 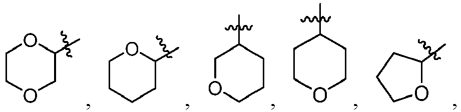 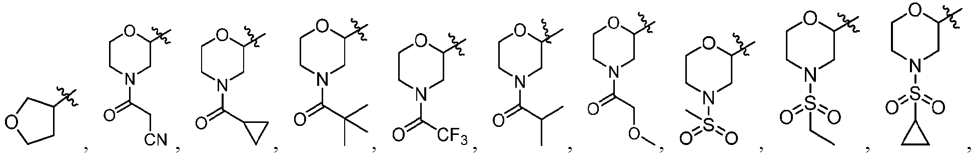 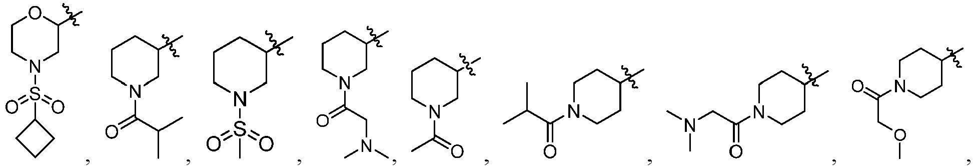 and 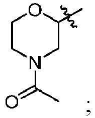
preferably, R³ is selected from the group consisting of

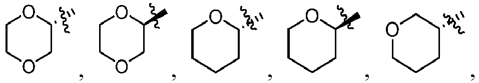

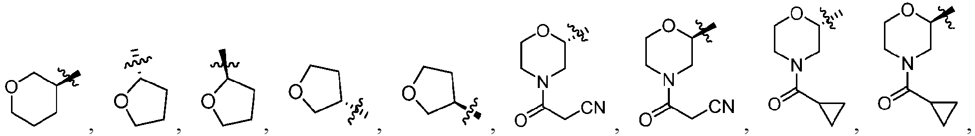

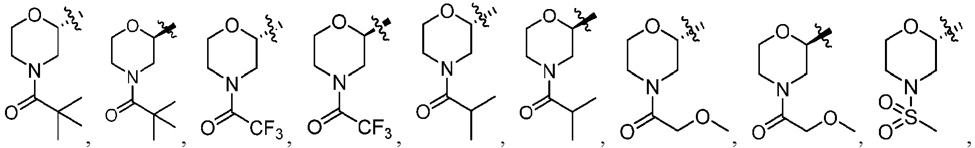

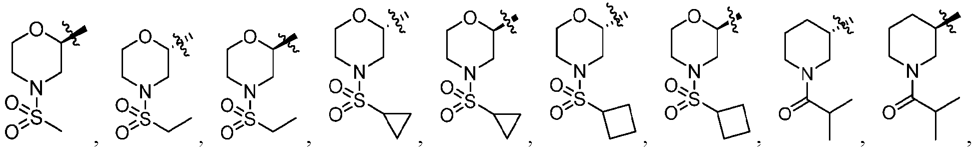

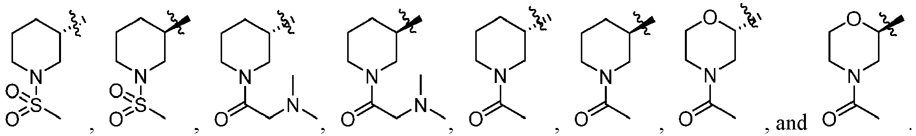

8. The compound of formula I, the stereoisomer thereof or the pharmaceutically acceptable salt thereof according to any one of claims 1 to 7, wherein G¹ is selected from C₁₋₆ alkyl substituted with OP(O)(OH)₂;
or G¹ is selected from C₁₋₄ alkyl substituted with OP(O)(OH)₂;
or G¹ is selected from C₁₋₃ alkyl substituted with OP(O)(OH)₂;
or G¹ is selected from -CH₂OP(O)(OH)₂.

9. The compound of formula I, the stereoisomer thereof or the pharmaceutically acceptable salt thereof according to any one of claims 1 to 8, being selected from the group consisting of a compound of formula II or formula III, a stereoisomer thereof and a pharmaceutically acceptable salt thereof: 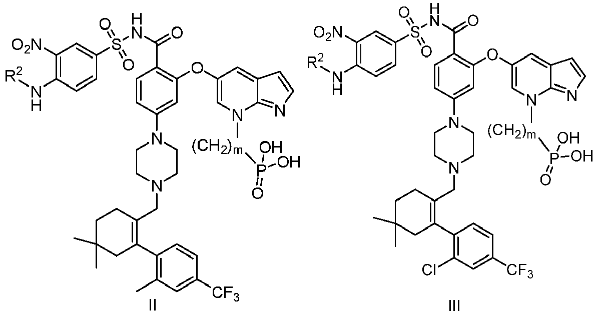 wherein R² is defined as in any one of claims 1 and 4 to 7; and m is selected from the group consisting of 1, 2, 3, and 4.

10. A compound of a formula selected from the group consisting of the following formulas, a stereoisomer thereof or a pharmaceutically acceptable salt thereof: 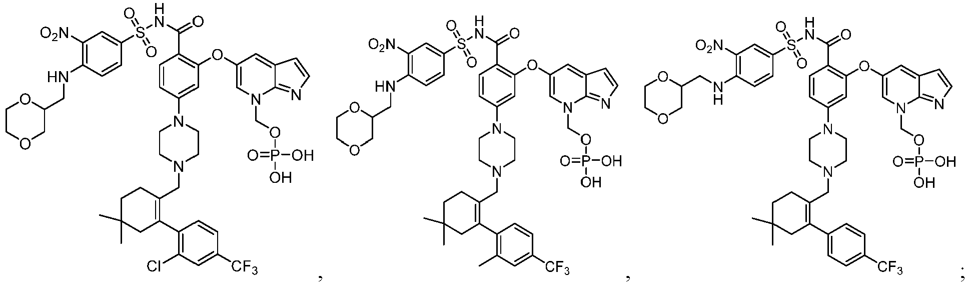 and, 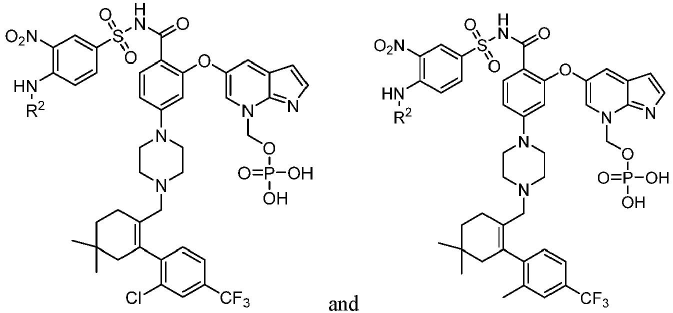
| **R²** | **R²** | **R²** | **R²** | **R²** | **R²** |
|---|---|---|---|---|---|
| 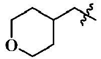 | 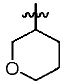 | 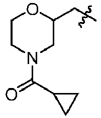 | 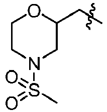 | 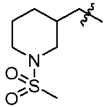 | 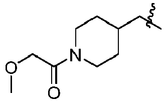 |
| 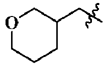 | 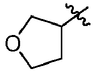 | 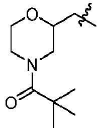 | 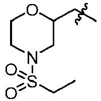 | 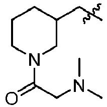 | 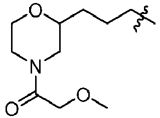 |
| 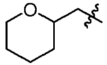 | 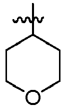 | 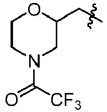 | 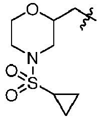 | 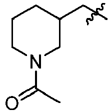 | 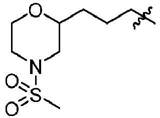 |
| 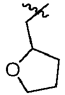 | 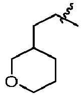 | 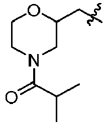 | 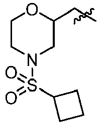 | 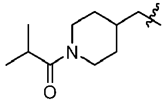 | 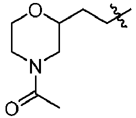 |
| 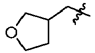 | 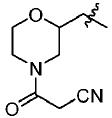 | 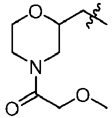 | 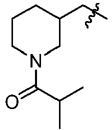 | 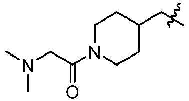 | |

11. The compound, the stereoisomer thereof or the pharmaceutically acceptable salt thereof according to claim 10, being selected from the group consisting of the following compounds, stereoisomers thereof and pharmaceutically acceptable salts thereof: 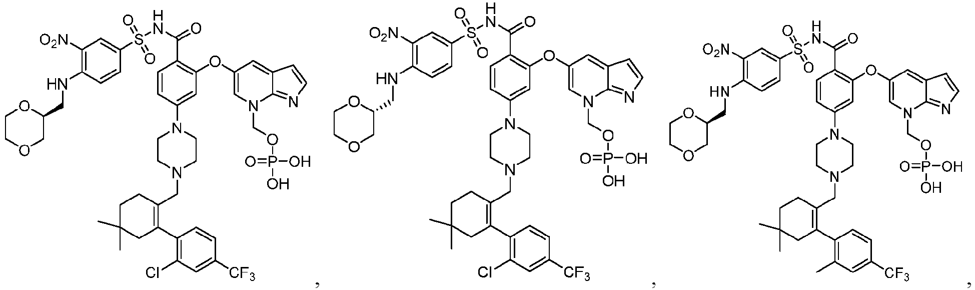 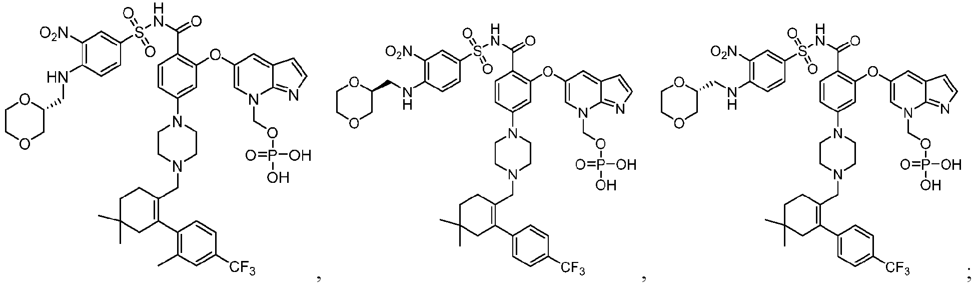 and, 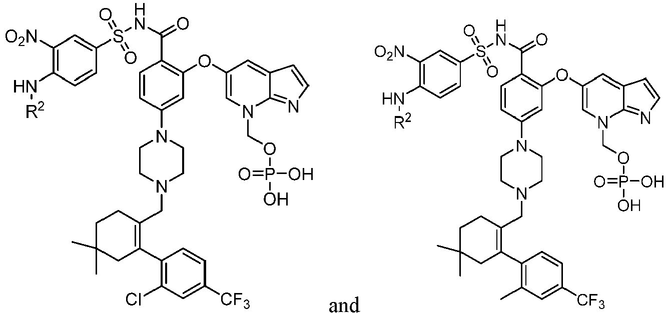
| **R²** | **R²** | **R²** | **R²** | **R²** | **R²** | **R²** | **R²** |
|---|---|---|---|---|---|---|---|
| 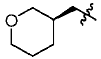 | 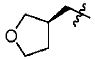 | 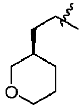 | 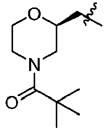 | 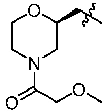 | 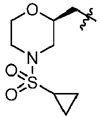 | 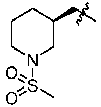 | 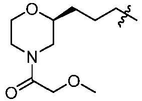 |
| 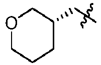 | 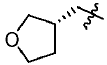 | 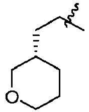 | 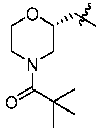 | 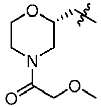 | 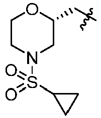 | 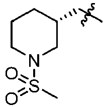 | 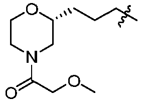 |
| 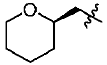 | 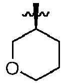 | 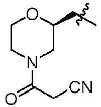 | 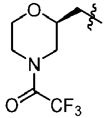 | 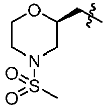 | 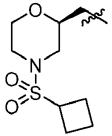 | 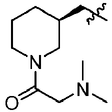 | 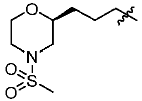 |
| 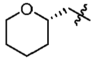 | 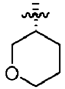 | 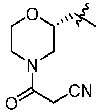 | 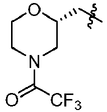 | 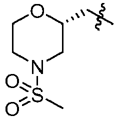 | 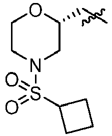 | 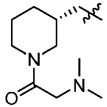 | 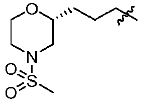 |
| 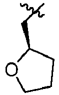 | 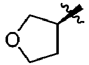 | 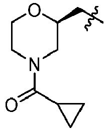 | 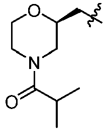 | 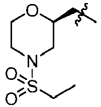 | 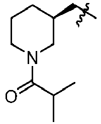 | 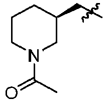 | 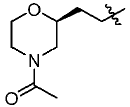 |
| 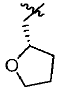 | 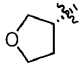 | 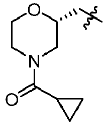 | 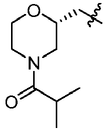 | 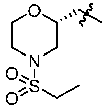 | 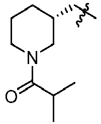 | 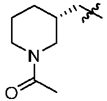 | 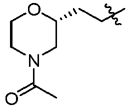 |

12. A pharmaceutical composition, comprising the compound, the stereoisomer thereof or the pharmaceutically acceptable salt thereof according to any one of claims 1 to 11.

13. Use of the compound, the stereoisomer thereof or the pharmaceutically acceptable salt thereof according to any one of claims 1 to 11, or the pharmaceutical composition according to claim 12 for preparing a medicament for preventing or treating an anti-apoptotic protein BCL-2-related disease,
wherein preferably, the anti-apoptotic protein BCL-2-related disease is a cancer; and
preferably, the cancer is acute lymphocytic leukemia.
